# EUROPÄISCHE PATENTSCHRIFT

(11) **EP 2 183 218 B1**
(45) Veröffentlichungstag und Bekanntmachung des Hinweises auf die Patenterteilung: **08.10.2014**
(21) Anmeldenummer: 08786337.9
(22) Anmeldetag: 23.07.2008
(51) Int. Cl.: C07D 209/40, C07D 209/44, C07D 211/58, C07D 217/06, C07D 223/16, C07D 401/14, C07D 471/10, C07D 487/04, A61K 31/4025, A61K 31/4035, A61K 31/4545, A61K 31/472, A61K 31/55, A61K 31/407, A61P 25/00, A61P 29/00

(54) **NEUE B1-ANTAGONISTEN**
NOVEL B1 ANTAGONISTS
NOUVEAUX ANTAGONISTES DE B1

(30) Priorität: 25.07.2007 DE 102007034620
(43) Veröffentlichungstag der Anmeldung: 12.05.2010
(73) Patentinhaber: Boehringer Ingelheim International GmbH, 55216 Ingelheim am Rhein (DE)
(72) Erfinder: KAUFFMANN-HEFNER, Iris, 55216 Ingelheim am Rhein (DE); CECI, Angelo, 55216 Ingelheim am Rhein (DE); DOODS, Henri, 55216 Ingelheim am Rhein (DE); HAUEL, Norbert, 55216 Ingelheim am Rhein (DE); KONETZKI, Ingo, 55216 Ingelheim am Rhein (DE); SCHULER-METZ, Annette, 55216 Ingelheim am Rhein (DE); WALTER, Rainer, 55216 Ingelheim am Rhein (DE)
(74) Vertreter: Simon, Elke Anna Maria
(86) Internationale Anmeldenummer: PCT/EP2008/059622
(87) Internationale Veröffentlichungsnummer: WO 2009/013299

(56) Entgegenhaltungen:
- WO-A-2008/046753
- US-A1- 2006 100 219
- US-A1- 2006 178 360
- DATABASE CAPLUS [Online] CHEMICAL ABSTRACTS SERVICE, COLUMBUS, OHIO, US; KRAVCHENKO, D. V. ET AL: "Synthesis of substituted 4-oxo-7-sulfamoyl-2,3,4,5- tetrahydrobenzo[b][1,4]thiazepines" XP002505852 gefunden im STN Database accession no. 2005:1039508 & IZVESTIYA VYSSHIKH UCHEBNYKH ZAVEDENII, KHIMIYA I KHIMICHESKAYA TEKHNOLOGIYA , 48(5), 123-131 CODEN: IVUKAR; ISSN: 0579-2991, 2005,
- MARCEAU F: "A possible common pharmacophore in the non-peptide antagonists of the bradykinin B1 receptor" TRENDS IN PHARMACOLOGICAL SCIENCES, ELSEVIER, HAYWARTH, GB, Bd. 26, Nr. 3, 1. März 2005 (2005-03-01), Seiten 116-118, XP004771633 ISSN: 0165-6147

## Beschreibung

Gegenstand der vorliegenden Erfindung sind Verbindungen der allgemeinen Formel I 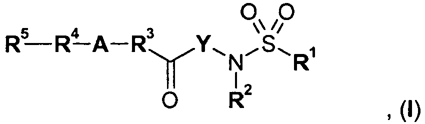 gemäß Anspruch 1 deren Enantiomere, deren Diastereomere, deren Gemische und deren Salze, insbesondere deren physiologisch verträgliche Salze mit organischen oder anorganischen Säuren oder Basen, welche wertvolle Eigenschaften aufweisen, deren Herstellung, die die pharmakologisch wirksamen Verbindungen enthaltenden Arzneimittel, deren Herstellung und deren Verwendung.

Substituierte Piperidine oder Piperazine als Antagonisten des Bradykinin B1 Rezeptors sind etwa aus der US2006/0178360 bekannt.

### DETAILLIERTE BESCHREIBUNG DER ERFINDUNG

Die vorliegende Erfindung betrifft Verbindungen, nämlich

| **Beispiel** | **Struktur** |
|---|---|
| (1) | 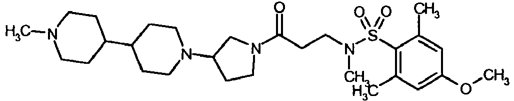 |
| (2) | 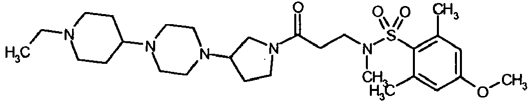 |
| (3) | 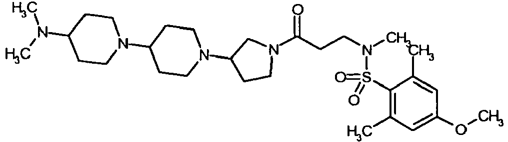 |
| (4) | 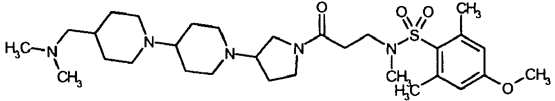 |
| (5) | 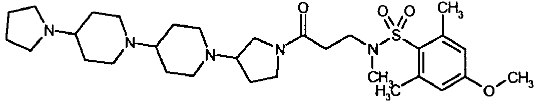 |
| (6) | 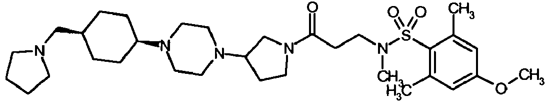 |
| (7) | 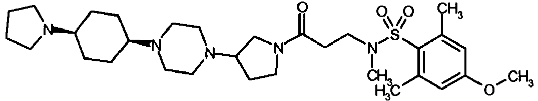 |
| (8) | 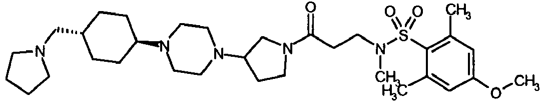 |
| (9) | 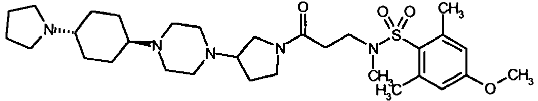 |
| (10) | 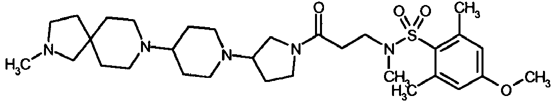 |
| (11) | 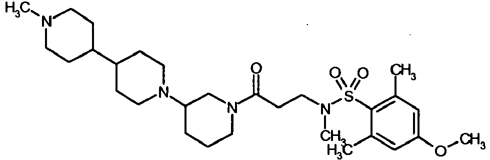 |
| (12) | 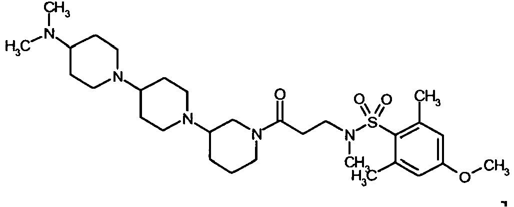 |

deren Enantiomere, deren Diastereomere, deren Gemische und deren Salze, insbesondere deren physiologisch verträgliche Salze mit organischen oder anorganischen Säuren oder Basen.

### VERWENDETE BEGRIFFE UND DEFINITIONEN

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Verbindungen der allgemeinen Formel I können, sofern sie geeignete basische Funktionen enthalten, beispielsweise Aminogruppen, insbesondere für pharmazeutische Anwendungen in ihre physiologisch verträglichen Salze mit anorganischen oder organischen Säuren übergeführt werden. Als anorganische Säuren kommen hierfür beispielsweise Bromwasserstoffsäure, Phosphorsäure, Salpetersäure, Salzsäure, Schwefelsäure, Methansulfonsäure, Ethansulfonsäure, Benzolsulfonsäure oder p-Toluolsulfonsäure in Frage, als organische Säuren kommen beispielsweise Äpfelsäure, Bernsteinsäure, Essigsäure, Fumarsäure, Maleinsäure, Mandelsäure, Milchsäure, Weinsäure oder Zitronensäure in Betracht.
Weiterhin lassen sich die Verbindungen der allgemeinen Formel I, sofern sie geeignete Carbonsäurefunktionen enthalten, gewünschtenfalls in ihre Additionssalze mit anorganischen oder organischen Basen überführen. Als anorganische Basen kommen hierfür beispielsweise Alkali- oder Erdalkalimetallhydroxide, beispielsweise Natriumhydroxid oder Kaliumhydroxid, oder Carbonate, Ammoniak, Zink- oder Ammoniumhydroxide in Frage; als organische Amine kommen beispielsweise Diethylamin, Triethylamin, Ethanolamin, Diethanolamin, Triethanolamin, Cyclohexylamin oder Dicyclohexylamin in Betracht.
Die erfindungsgemäßen Verbindungen können als Racemate vorliegen, sofern sie nur ein Chiralitätselement besitzen, sie können aber auch als reine Enantiomere, d.h. in (*R*)- oder (*S*)-Form gewonnen werden.
Die Anmeldung umfasst jedoch auch die einzelnen diastereomeren Antipodenpaare oder deren Gemische, die dann vorliegen, wenn mehr als ein Chiralitätselement in den Verbindungen der allgemeinen Formel I vorhanden ist, sowie die einzelnen optisch aktiven Enatiomeren, aus denen sich die erwähnten Racemate zusammensetzen.

### Methodenbeschreibung zur hBK1-Rezeptorbindung

CHO-Zellen, die den hBK1-Rezeptor exprimieren, werden in "Dulbecco's modified Medium" kultiviert. Von konfluenten Kulturen wird das Medium entfernt, die Zellen werden mit PBS-Puffer gewaschen, abgeschabt und durch Zentrifugieren isoliert. Anschließend werden die Zellen in Suspension homogenisiert, das Homogenat zentrifugiert und resuspendiert. Nach Bestimmung des Proteingehalts wird die so erhaltene Membranpräparation bei -80°C eingefroren.

Nach dem Auftauen werden 200 µl des Homogenats (50 bis100 µg Protein/Assay) für 60 Minuten bei Raumtemperatur mit 0.5 bis 1.0 nM Kallidin (DesArg10,Leu9), [3,4-Prolyl-3,43H(N)] und ansteigenden Konzentrationen der Testsubstanz in einem Gesamtvolumen von 250 µl inkubiert. Die Inkubation wird durch rasche Filtration durch GF/B-Glasfaserfilter, die mit Polyethyleneimine (0.3%) vorbehandelt wurden, beendet. Die an das Protein gebundene Radioaktivität wird mit einem TopCount NXT gemessen. Als nichtspezifische Bindung wird die gebundene Radioaktivität in Gegenwart von 1.0 µM Kallidin (DesArg10,Leu9), [3,4-Prolyl-3,43H(N)] definiert. Die Analyse der Konzentrations-Bindungskurve erfolgt mit Hilfe einer computergestützten nichtlinearen Kurvenanpassung. Aus den so erhaltenen Daten wird für die Testsubstanz der entsprechende Kᵢ - Wert ermittelt.

### INDIKATIONEN

Aufgrund ihrer pharmakologischen Eigenschaften eignen sich die neuen Verbindungen und deren physiologisch verträglichen Salze zur Behandlung von Krankheiten und Krankheitssymptomen, die wenigstens teilweise durch die Stimulierung von Bradykinin-B1-Rezeptoren hervorgerufen werden.
Aufgrund ihrer pharmakologischen Wirkung eignen sich die Substanzen zur Behandlung
(a) von **akuten Schmerzen** wie z.B. Zahnschmerzen, peri- und postoperativen Schmerzen, traumatischen Schmerzen, Muskelschmerzen, Verbrennungsschmerzen, Schmerzen bei Sonnenbrand, Trigeminusneuralgie, Schmerzen bei Koliken, sowie bei Spasmen des Magen-Darm-Trakts oder des Uterus;
(b) von **Eingeweideschmerzen** wie z.B. chronischen Beckenschmerzen, gynäkologischen Schmerzen, Schmerzen vor und während der Menstruation, Schmerzen bei Pankreatitis, bei peptischen Ulzera, bei interstitieller Zystitis, bei Nierenkolik, bei Angina pectoris, Schmerzen bei Kolon irritabile, bei nicht-ulzeröser Dyspepsie und bei Gastritis, nicht-kardialen Thoraxschmerzen und Schmerzen bei myokardialer Ischämie und Herzinfarkt;
(c) von **neuropathischen Schmerzen** wie z.B. schmerzhaften Polyneuropathien, Schmerzen bei diabetischer Neuropathie, AIDS-assoziierten neuropathischen Schmerzen, Schmerzen bei Lumbago, bei nicht-herpesassoziierter Neuralgie, bei Post-zoster Neuralgie, bei Nervenverletzungen, bei Schädel-Hirn-Trauma, Schmerzen bei Nervenchädigungen infolge von Toxinen oder Chemotherapie, Phantomschmerzen, Schmerzen bei multipler Sklerose, bei Nervenwurzelabriß und schmerzhaften traumatisch bedingten Einzelnervenschädigungen;
(d) von **entzündlichen / Schmerzrezeptor-vermittelten Schmerzen** im Zusammenhang mit Erkrankungen wie Osteoarthritis, rheumatoider Arthritis, rheumatischem Fieber, Tendo-Synovitis, Sehnenentzündungen, Gicht, Vulvodynie, Schädigungen und Erkrankungen der Muskeln und Faszien (muskuläre Verletzungen, Fibromyalgie), Osteoarthritis, juveniler Arthritis, Spondylitis, Gicht-Arthritis, Psoriasis-Arthritis, Fibromyalgie, Myositis, Migräne, Zahnerkrankungen, Influenza und anderen Virusinfektionen wie Erkältungen, systemischer Lupus erythematodes,
(e) von **Tumorschmerzen** im Zusammenhang mit Krebserkrankungen wie lymphatischer oder myeloischer Leukämie, Hodgkin Erkrankung, Non-Hodgkin Lymphomen, Lymphogranulomatose, Lymphosarkomen, soliden malignen Tumoren und ausgedehnten Metastasen;
(f) von **Kopfschmerz-Erkrankungen** wie z.B. Kopfschmerzen unterschiedlicher Ursache, Cluster-Kopfschmerz, Migräne (mit oder ohne Aura) und Spannungskopfschmerz.
   Weiterhin eigenen sich die Verbindungen zur Behandlung
(g) von entzündlichen Veränderungen im Zusammenhang mit Erkrankungen der Atemwege wie Asthma bronchiale, einschließlich allergischem Asthma (atopisch und nicht-atopisch) sowie Bronchospasmen bei Anstrengung, beruflich-bedingtem Asthma, viraler oder bakterieller Exazerbation einer bestehenden Asthma-Erkrankung und anderen nicht-allergisch bedingten asthmatischen Erkrankungen;
   chronisch obstruktiver Lungen-Erkrankung (COPD) einschließlich Lungenemphysem,
   akutem Atemnotsyndrom des Erwachsenen (ARDS), Bronchitis, Lungenentzündung, allergischer Rhinitis (saisonal und ganzjährig), vasomotorischer Rhinitis und Staublungen-Erkrankungen wie Aluminose, Anthrakose, Asbestose, Chalikose, Siderose, Silikose, Tabakose und Byssinose;
(h) von Entzündungserscheinungen bei Sonnenbrand und Verbrennungen, Ödemen nach Traumata durch Verbrennungen, Hirnödemen und Angioödemen, Darmerkrankungen einschließlich Morbus Crohn und Kolitis ulzerosa, Reizdarmsyndrom, Pankreatitis, Nephritis, Zystitis (interstitielle Zystitis), Uveitis; entzündlichen Erkrankungen der Haut (wie z.B. Psoriasis und Ekzeme), vaskulären Bindegewebserkrankungen, Lupus, Zerrungen und Frakturen;
(i) von Diabetes Mellitus und dessen Folgen (wie z.B. diabetische Vaskulopathie, diabetische Neuropathie, diabetische Retinopathie) und von diabetischen Symptomen bei Insulitis (z.B. Hyperglycämie, Diurese, Proteinurie und erhöhte renale Nitrit- und Kallikrein-Exkretion);
(j) von neurodegenerativen Erkrankungen wie der Parkinson'schen Erkrankung und der Alzheimer Erkrankung;
(k) von Sepsis und septischem Schock nach bakteriellen Infektionen oder nach Trauma;
(l) von Juckreiz verursachenden Syndromen und allergischen Hautreaktionen;
(m) von Osteoporose;
(n) von Epilepsie;
(o) von Verletzungen des Zentralnervensystems;
(p) von Wunden und Gewebeschädigungen;
(q) von Zahnfleischentzündungen;
(r) von benigner Prostata-Hyperplasie und hyperaktiver Blase;
(s) von Pruritus;
(t) von Vitiligo;
(u) von Störungen der Motilität von respiratorischen, genito-urinalen, gastro-intestinalen oder vaskulären Regionen und
(v) von post-operativem Fieber.

Neben der Eignung als Humantherapeutika, sind diese Substanzen auch nützlich in der veterinärmedizinischen Therapie von Haustieren, exotischen Tieren und Nutztieren.

### KOMBINATIONEN

Zur Behandlung von Schmerzen kann es vorteilhaft sein, die erfindungsgemäßen Verbindungen mit belebenden Stoffen wie Koffein oder anderen schmerzlindernden Wirkstoffen zu kombinieren. Stehen zur Behandlung der Ursache der Schmerzen geeignete Wirkstoffe zur Verfügung, so können diese mit den erfindungsgemäßen Verbindungen kombiniert werden. Sind unabhängig von der Schmerzbehandlung auch noch weitere medizinische Behandlungen angezeigt, zum Beispiel gegen Bluthochdruck oder Diabetes, so können auch die dafür nötigen Wirkstoffe mit den erfindungsgemäßen Verbindungen kombiniert werden.

Für eine Kombinationstherapie kommen beispielsweise die folgenden Verbindungen in Frage:
Nicht-steroidale Antirheumatika (NSAR): COX-2 Hemmer wie Propionsäure-Derivate (Alminoprofen, Benoxaprofen, Bucloxinsäure, Carprofen, Fenhufen, Fenoprofen, Fiuprofen, Fiulbiprofen, Ibuprofen, Indoprofen, Ketoprofen, Miroprofen, Naproxen, Oxaprozin, Pirprofen, Pranoprofen, Suprofen, Tiaprofensäure, Tioxaprofen), Essigsäure-Derivate (Indomethacin, Acemetacin, Alcofenac, Isoxepac, Oxpinax, Sulindac, Tiopinac, Tolmetin, Zidometacin, Zomepirac) Fenaminsäure-Derivate (Meclofenaminsäure, Mefenaminsäure, Tolfenaminsäure), Biphenyl-Carboxylsäure-Derivate, Oxicame (Isoxicam, Meloxicam, Piroxicam, Sudoxicam und Tenoxicam), Salicylsäure-Derivate (Acetylsalicylsäure, Sulfasalazin, warum nicht auch Mesalazin, Olsalazin, und Pyrazolone (Apazon, Bezpiperylon, Feprazon, Mofebutazon, Oxyphenbutazon, Phenylbutazon, warum nicht auch Propyphenazon und Metamizol, und Coxibe (Celecoxib, Valecoxib, Rofecoxib, Etoricoxib).
Opiat Rezeptor Agonisten wie z. B. Morphin, Propoxyphen (Darvon), Tramadol, Buprenorphin. Cannabinoid Agonisten wie z.B. GW-1000, KDS-2000, SAB-378, SP-104, NVP001-GW-843166, GW-842166X, PRS-211375.
Natriumkanalblocker wie z.B. Carbamazepin, Mexiletin, Lamotrigin, Pregabalin, Tectin, NW-1029, CGX-1002.
N-Typ Calciumkanalblocker wie z.B. Ziconitid, NMED-160, SP1-860.
Serotonerge und noradrenerge Modulatoren wie z.B. SR-57746, Paroxetin, Duloxetin, Clonidin, Amitriptylin, Citalopram.
Corticosteroide wie z.B. Betamethason, Budesonid, Cortison, Dexamethason, Hydrocortison, Methylprednisolon, Prednisolon, Prednison und Triamcinolon.
Histamin H1-Rezeptorantagonisten wie z.B. Bromophtniramint, Chlorpheniramin, Dexchlorpheniramin, Triprolidin, Clemastin, Diphenhydramin, Diphenylpyralin, Tripelennamin, Hydroxyzin, Methdijazin, Promethazin, Trimeprazin Azatadin, Cyproheptadin, Antazolin, Pheniramin, Pyrilamin, Astemizol, Terfenadin, Loratadin, Cetirizin, Desloratadin, fexofenadin, Levocetirizin.
Histamin H2-Rezeptor Antagonisten wie z.B. Cimetidin, Famotidin, und Ranitidin. Protonenpumpenhemmer wie z.B. Omeprazol, Pantoprazol, Esomeprazol.
Leukotrien Antagonisten und 5-Lipoxygenasehemmer wie z.B. Zafirlukast, Montelukast, Pranlukast und Zileuton.
Lokalanästhetika wie z.B. Ambroxol, Lidocain.
VR1 Agonisten und Antagonisten wie z.B. NGX-4010, WL-1002, ALGRX-4975, WL-10001, AMG-517.
Nikotinrezeptor Agonisten wie z.B. ABT-202, A-366833, ABT-594, BTG-102, A-85380, CGX1204.
P2X3-Rezeptor Antagonisten wie z.B. A-317491, ISIS-13920, AZD-9056.
NGF Agonisten und Antagonisten wie z.B. RI-724, RI-1024, AMG-819, AMG-403, PPH 207. NK1 und NK2 Antagonisten wie z.B. DA-5018, R-116301, CP-728663, ZD-2249.
NMDA Antagonisten wie z.B. NER-MD-11, CNS-5161, EAA-090, AZ-756, CNP-3381. Kaliumkanal Modulatoren wie z.B. CL-888, ICA-69673, Retigabin.
GABA Modulatoren wie z.B. Lacosamid.
Serotonerge und noradrenerge Modulatoren wie z.B. SR-57746, Paroxetin, Duloxetin, Clonidin, Amitriptylin, Citalopram, Flibanserin.
Anti-Migräne Therapeutika wie z.B. Sumatriptan, Zolmitriptan, Naratriptan, Eletriptan.

Die zur Erzielung einer schmerzstillenden Wirkung erforderliche Dosierung beträgt bei intravenöser Gabe zweckmäßigerweise 0.01 bis 3 mg/kg Körpergewicht, vorzugsweise 0.1 bis 1 mg/kg, und bei oraler Gabe 0.1 bis 8 mg/kg Körpergewicht, vorzugsweise 0.5 bis 3 mg/kg, jeweils 1 bis 3 x täglich. Die erfindungsgemäß hergestellten Verbindungen können intravenös, subkutan, intramuskulär, intrarektal, intranasal, durch Inhalation, transdermal oder oral verabreicht werden, wobei zur Inhalation insbesondere Aerosolformulierungen geeignet sind. Sie können gegebenenfalls zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen, Lösungen, Dosieraerosole oder Zäpfchen eingearbeitet werden.

### EXPERIMENTELLER TEIL

Für die hergestellten Verbindungen liegen in der Regel IR-, ¹H-NMR und/oder Massenspektren vor. Die bei den Fliessmitteln angegebenen Verhältnisse beziehen sich auf Volumeneinheiten der jeweiligen Lösungsmittel. Die angegebenen Volumeneinheiten bei Ammoniak beziehen sich auf eine konzentrierte Lösung von Ammoniak in Wasser.
Soweit nicht anders vermerkt sind die bei den Aufarbeitungen der Reaktionslösungen verwendeten Säure-, Basen- und Salzlösungen wässrige Systeme der angegebenen Konzentrationen.
Zu chromatographischen Reinigungen wird Kieselgel der Firma Millipore (MATREX^{™}, 35-70 µm) oder Alox (E. Merck, Darmstadt, Aluminiumoxid 90 standardisiert, 63-200 µm, Artikel-Nr: 1.01097.9050) verwendet.

In den Versuchsbeschreibungen werden die folgenden Abkürzungen verwendet:
- DC: Dünnschichtchromatogramm
- DIPEA: Diisopropylethylamin
- DMF: Dimethylformamid
- HPLC: High Presssure Liquid Chromatographie
- *tert*: tertiär
- TBTU: 2-(1*H*-Benzotriazol-1-yl)-1,1,3,3-tetramethyluronium-tetrafluorborat
- TFA: Trifluoressigsäure
- THF: Tetrahydrofuran

Folgende analytische HPLC-Methoden wurden verwendet:
Methode 1:

| | | | |
|---|---|---|---|
| Säule: | YMC-Pack ODS-AQ, 3.0 µM, 4.6 x 75 mm | | |
| Detektion: | 230 - 360 nm | | |
| Fließmittel A: | Wasser / 0.1 % Ameisensäure | | |
| Fließmittel B: | Acetonitril / 0.1 % Ameisensäure | | |
| Gradient: | | | |

| Zeit in min | %A | %B | Flussrate in mL/min |
|---|---|---|---|
| 0.0 | 95.0 | 5.0 | 1.6 |
| 4.5 | 10.0 | 90.0 | 1.6 |
| 5.0 | 10.0 | 90.0 | 1.6 |
| 5.5 | 90.0 | 10.0 | 1.6 |

Methode 2:

| | | | |
|---|---|---|---|
| Säule: | Merck Chromolith™ Flash RP18e, 4.6 x 25 mm | | |
| Detektion: | 190 - 400 nm | | |
| Fließmittel A: | Wasser / 0.1 % Ameisensäure | | |
| Fließmittel B: | Acetonitril / 0.1 % Ameisensäure | | |
| Gradient: | | | |

| Zeit in min | %A | %B | Flussrate in mL/min |
|---|---|---|---|
| 0.0 | 90.0 | 10.0 | 1.6 |
| 2.7 | 10.0 | 90.0 | 1.6 |
| 3.0 | 10.0 | 90.0 | 1.6 |
| 3.3 | 90.0 | 10.0 | 1.6 |

Methode 3:

| | | | |
|---|---|---|---|
| Säule: | Zorbax Stable Bond C18, 3.5 µM, 4.6 x 75 mm | | |
| Detektion: | 230 - 360 nm | | |
| Fließmittel A: | Wasser / 0.1 % Ameisensäure | | |
| Fließmittel B: | Acetonitril / 0.1 % Ameisensäure | | |
| Gradient: | | | |

| Zeit in min | %A | %B | Flussrate in mL/min |
|---|---|---|---|
| 0.0 | 95.0 | 5.0 | 1.6 |
| 0.1 | 95.0 | 5.0 | 1.6 |
| 4.5 | 10.0 | 90.0 | 1.6 |
| 5.09 | 10.0 | 90.0 | 1.6 |
| 5.5 | 90.0 | 10.0 | 1.6 |

Methode 4:

| | | | |
|---|---|---|---|
| Säule: | Interchim Strategy C18, 5 µM, 4.6 x 50 mm | | |
| Detektion: | 220 - 320 nm | | |
| Fließmittel A: | Wasser / 0.1 % TFA | | |
| Fließmittel B: | Acetonitril | | |
| Gradient: | | | |

| Zeit in min | %A | %B | Flussrate in mL/min |
|---|---|---|---|
| 0.0 | 95.0 | 5.0 | 3.0 |
| 0.3 | 95.0 | 5.0 | 3.0 |
| 2.0 | 2.0 | 98.0 | 3.0 |
| 2.4 | 2.0 | 98.0 | 3.0 |
| 2.45 | 95.0 | 5.0 | 3.0 |
| 2.8 | 95.0 | 5.0 | 3.0 |

Methode 5:

| | | | |
|---|---|---|---|
| Säule: | Waters XBridge C18, 3.5 µM, 4.6 x 50 mm | | |
| Detektion: | 210 - 500 nm | | |
| Fließmittel A: | Wasser / 0.1 % TFA | | |
| Fließmittel B: | Acetonitril / 0.1 % TFA | | |
| Gradient: | | | |

| Zeit in min | %A | %B | Flussrate in mL/min |
|---|---|---|---|
| 0.0 | 95.0 | 5.0 | 1.5 |
| 2.0 | 0.0 | 100.0 | 1.5 |
| 3.0 | 0.0 | 100.0 | 1.5 |
| 3.4 | 95.0 | 5.0 | 1.5 |

### Herstellung der Endverbindungen

### Beispiel 1

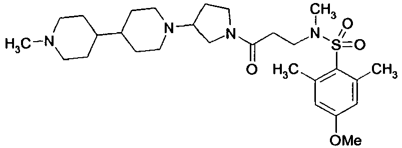

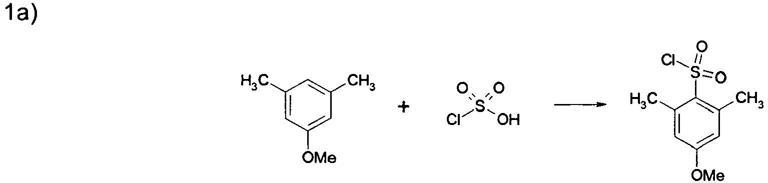

Eine Mischung aus 2.0 g (14.69 mMol) 3,5-Dimethylanisol und 20 ml Dichlormethan wird unter Eisbadkühlung mit 5.85 ml (88.0 mMol) Chlorsulfonsäure versetzt. Die Reaktionsmischung wird danach 20 min bei Raumtemperatur gerührt und anschließend auf 50 ml Eiswasser gegossen. Man extrahiert mit 100 ml Dichlormethan. Die organischen Extrakte werden mit 5%iger Natriumhydrogencarbonat-Lösung gewaschen, über Natriumsulfat getrocknet und bis zur Trockene eingeengt.
C₉H₁₁ClO₃S (234.70)
[M+H]+ = 234/236
DC: Kieselgel, Petrolether / Ethylacetat 9:1, Rf-Wert = 0.46

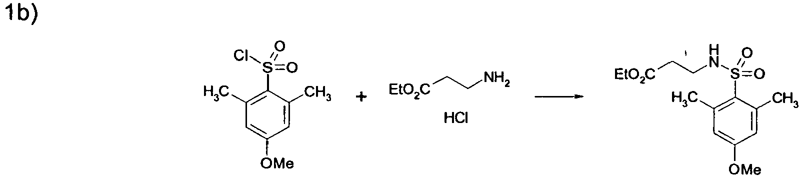

Eine Mischung aus 3.00 g (12.78 mMol) Produkt aus 1a, 2.16 g (14.06 mMol) ß-Alaninethylester Hydrochlorid, 7.13 ml (51.13 mMol) Triethylamin und 70 ml Dichlormethan wird über Nacht bei Raumtemperatur gerührt. Die Reaktionsmischung wird anschließend mit 0.5 M HCl, gesättigter Natriumhydrogencarbonatlösung, Wasser und gesättigter Natriumchloridlösung gewaschen, über Natriumsulfat getrocknet und im Vakuum bis zur Trockene eingeengt.
C₁₄H₂₁NO₅S (315.39); [M+H]+ = 316
DC: Kieselgel, Petrolether / Ethylacetat 2:1, Rf-Wert = 0.23

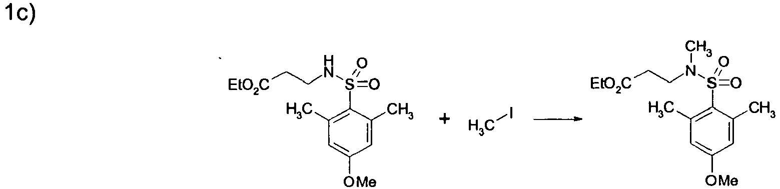

Eine Mischung aus 4.06 g (12.87 mMol) Produkt aus 1 b, 0.84 ml (13.55 mMol) Methyliodid, 3.56 g (25.75 mMol) Kaliumcarbonat wasserfrei und 40 ml DMF wird fünf Stunden bei Raumtemperatur gerührt. Die Reaktionsmischung wird anschließend im Vakuum bis zur Trockene eingeengt, der Rückstand wird in Ethylacetat aufgenommen. Man wäscht mit Wasser, gesättigter Natriumhydrogencarbonatlösung und gesättigter Natriumchloridlösung, trocknet über Natriumsulfat und engt im Vakuum bis zur Trockene ein.
C₁₅H₂₃NO₅S (329.41)
[M+H]+ = 330
DC: Kieselgel, Petrolether / Ethylacetat 2:1, Rf-Wert = 0.36

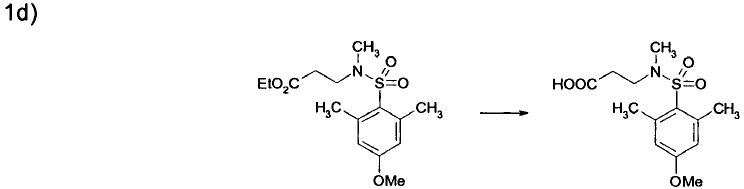

Eine Mischung aus 3.83 g (11.63 mMol) Produkt aus 1c, 2.44 g (58.13 mMol) Lithiumhydroxid Monohydrat, 30 ml THF und 30 ml Wasser wird eine Stunde bei Raumtemperatur gerührt. Danach wird das THF im Vakuum entfernt und der Rückstand mit konzentrierter HCl sauer gestellt. Die Reaktionsmischung wird anschließend dreimal mit Ethylacetat extrahiert. Die organischen Extrakte werden mit gesättigter Natriumchloridlösung gewaschen, über Natriumsulfat getrocknet und zur Trockne eingeengt. Das Rohprodukt wird mit Diethylether verrieben und abgesaugt.
C₁₃H₁₉NO₅S (301.36)
[M+H]+ = 302
DC: Kieselgel, Petrolether / Ethylacetat 1:1, Rf-Wert = 0.12

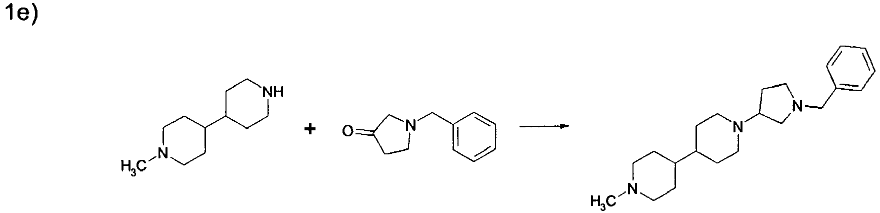

Eine Mischung aus 1.19 g (6.60 mMol) 1-Methyl-[4,4']bipiperidin (J. Chem. Soc. 1957, 3165-3172), 1.28 g (7.80 mMol) 1-Benzyl-3-pyrrolidinon (Aldrich) und 60 ml THF wird eine Stunde bei Raumtemperatur gerührt. Danach werden 1.65 g (7.80 mMol) Natriumtriacetoxyborhydrid und 0.37 ml (6.50 mMol) Eisessig hinzugefügt. Die Reaktionsmischung wird anschließend über Nacht bei Raumtemperatur gerührt und dann mit halbgesättigter Kaliumcarbonat-Lösung versetzt. Das THF wird im Vakuum entfernt, der wässrige Rückstand mit Ethylacetat extrahiert. Die organischen Extrakte werden über Natriumsulfat getrocknet und zur Trockne eingeengt. Das so erhaltene Rohprodukt wird durch Säulenchromatographie (Elutionsmittel: Dichlormethan / Methanol / Ammoniak 95:5:0.5 bis 90:10:1) gereinigt.
C₂₂H₃₅N₃ (341.53)
[M+H]+ = 342
DC: Kieselgel, Dichlormethan / Methanol / Ammoniak 9:1:0.1, Rf-Wert = 0.31

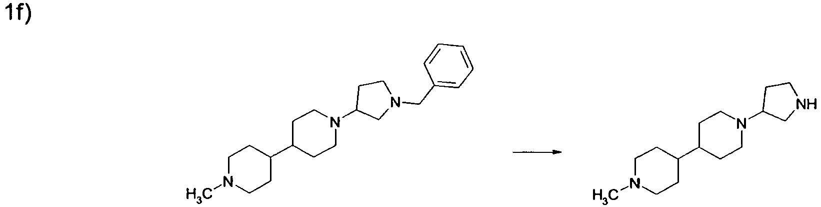

Eine Mischung aus 1.43 g (4.19 mMol) Produkt aus 1e, 0.30 g Palladiumhydroxid und 40 ml Methanol wird vier Stunden im Autoklaven bei Raumtemperatur hydriert. Anschließend wird der Katalysator abfiltriert und das Filtrat im Vakuum bis zur Trockene eingeengt.
C₁₅H₂₉N₃ (251.41)
[M+H]+ = 252
DC: Kieselgel, Dichlormethan / Methanol / Ammoniak 8:2:0.2, Rf-Wert = 0.08

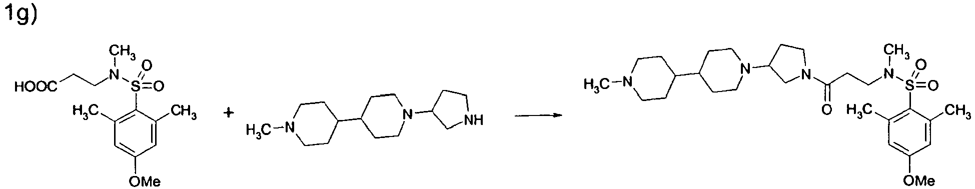

Eine Mischung aus 90.4 mg (0.30 mMol) Produkt aus 1d, 83.6 µl (0.60 mMol) Triethylamin, 106.0 mg (0.33 mMol) TBTU, 7 ml THF und 1 ml DMF wird 30 min bei Raumtemperatur gerührt. Danach werden 75.4 mg (0.30 mMol) Produkt aus 1f hinzugefügt und über Nacht weiter bei Raumtemperatur gerührt. Anschließend wird das THF im Vakuum entfernt, der DMFhaltige Rückstand mit 2 ml Methanol/Wasser und einigen Tropfen Ameisensäure versetzt. Diese Mischung wird über ein Spritzenfilter filtriert und danach mit Hilfe von präparativer HPLC das Produkt eluiert. Die entsprechenden Eluate werden bis zur Trockene eingeengt, mit gesättigter Kaliumcarbonat-Lösung versetzt und mit Ethylacetat extrahiert. Die organischen Extrakte werden über Natriumsulfat getrocknet und im Vakuum bis zur Trockene eingeengt. Das so erhaltene Produkt wird mit Diisopropylether verrieben und in der Trockenpistole bei 60°C getrocknet.
C₂₈H₄₆N₄O₄S (534.76)
[M+H]+ = 535
HPLC (Methode 1): Retentionszeit = 2.3 min

Folgende Verbindungen (Beispiel 2-12) wurden analog Beispiel 1 hergestellt:

### Beispiel 2

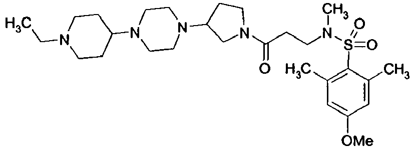

C₂₈H₄₇N₅O₄S (549.77)
[M+H]+ = 550
HPLC (Methode 1): Retentionszeit = 2.3 min

### Beispiel 3

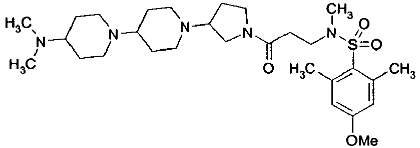

C₂₉H₄₉N₅O₄S (563.80)
[M+H]+ = 564
HPLC (Methode 1): Retentionszeit = 2.0 min

### Beispiel 4

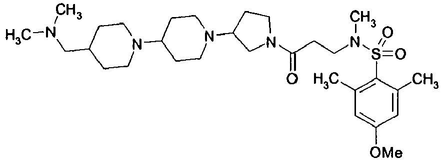

C₃₀H₅₁N₅O₄S (577.82)
[M+H]+ = 578
HPLC (Methode 1): Retentionszeit = 2.0 min

### Beispiel 5

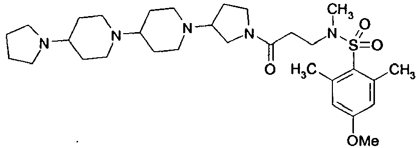

C₃₁H₅₁N₅O₄S (589.83)
[M+H]+ = 590
HPLC (Methode 1): Retentionszeit = 2.0 min

### Beispiel 6

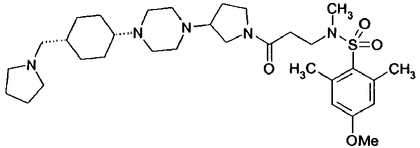

C₃₂H₅₃N₅O₄S x 3HCl (713.24)
[M+H]+ = 604
DC: Kieselgel, Dichlormethan / Methanol / Ammoniak 8:2:0.2, Rf-Wert = 0.71

### Beispiel 7

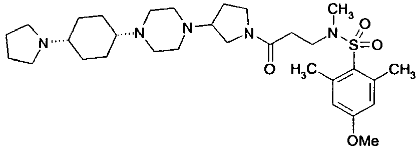

C₃₁H₅₁N₅O₄S x 3HCl (699.22)
[M+H]+ = 590
HPLC (Methode 1): Retentionszeit = 2.3 min

### Beispiel 8

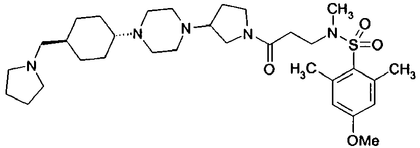

C₃₂H₅₃N₅O₄S (603.86)
[M+H]+ = 604
DC: Kieselgel, Dichlormethan / Methanol / Ammoniak 8:2:0.2, Rf-Wert = 0.64

### Beispiel 9

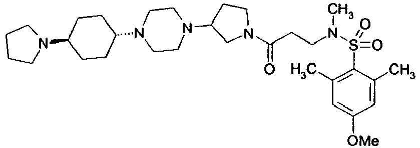

C₃₁H₅₁N₅O₄S (589.83)
[M+H]+ = 590
DC: Kieselgel, Dichlormethan / Methanol / Ammoniak 8:2:0.2, Rf-Wert = 0.49

### Beispiel 10

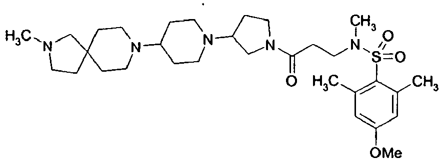

C₃₁H₅₁N₅O₄S x 3HCl (699.22)
[M+H]+ = 590
HPLC (Methode 1): Retentionszeit = 2.06 min

### Beispiel 11

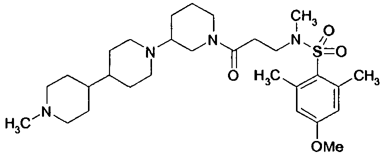

C₂₉H₄₈N₄O₄S x 2HCl (621.70)
[M+H]+ = 549
HPLC (Methode 1): Retentionszeit = 2.4 min

### Beispiel 12

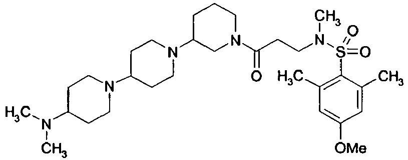

C₃₀H₅₁N₅O₄S x 3HCl (687.21)
[M+H]+ = 578
HPLC (Methode 1): Retentionszeit = 2.1 min

Die nachfolgenden Beispiele beschreiben pharmazeutischer Darreichungsformen, die als Wirkstoff eine beliebige Verbindung der allgemeinen Formel I enthalten:

### Beispiel I

Trockenampulle mit 75 mg Wirkstoff pro 10 ml

### Zusammensetzung:

| | |
|---|---|
| Wirkstoff | 75.0 mg |
| Mannitol | 500 mg |
| Wasser für Injektionszwecke ad | 10.0 ml |

### Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

### Beispiel II

### Tablette mit 50 mg Wirkstoff

### Zusammensetzung:

| | |
|---|---|
| (1) Wirkstoff | 50.0 mg |
| (2) Milchzucker | 98.0 mg |
| (3) Maisstärke | 50.0 mg |
| (4) Polyvinylpyrrolidon | 15.0 mg |
| (5) Magnesiumstearat | 2.0 mg |
| | 215.0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 9 mm.

### Beispiel III

Tablette mit 350 mg Wirkstoff

### Zusammensetzung:

| | |
|---|---|
| (1) Wirkstoff | 350.0 mg |
| (2) Milchzucker | 136.0 mg |
| (3) Maisstärke | 80.0 mg |
| (4) Polyvinylpyrrolidon | 30.0 mg |
| (5) Magnesiumstearat | 4.0 mg |
| | 600.0 mg |

### Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 12 mm.

### Beispiel IV

### Kapseln mit 50 mg Wirkstoff

### Zusammensetzung:

| | |
|---|---|
| (1) Wirkstoff | 50.0 mg |
| (2) Maisstärke getrocknet | 58.0 mg |
| (3) Milchzucker pulverisiert | 50.0 mg |
| (4) Magnesiumstearat | 2.0 mg |
| | 160.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 3 abgefüllt.

### Beispiel V

### Kapseln mit 350 mg Wirkstoff

### Zusammensetzung:

| | |
|---|---|
| (1) Wirkstoff | 350.0 mg |
| (2) Maisstärke getrocknet | 46.0 mg |
| (3) Milchzucker pulverisiert | 30.0 mg |
| (4) Magnesiumstearat | 4.0 mg |
| | 430.0 mg |

### Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Gr6Be 0 abgefüllt.

### Beispiel VI

### Suppositorien mit 100 mg Wirkstoff

### 1 Zäpfchen enthält:

| | |
|---|---|
| Wirkstoff | 100.0 mg |
| Polyethylenglykol (M.G. 1500) | 600.0 mg |
| Polyethylenglykol (M.G. 6000) | 460.0 mg |
| Polyethylensorbitanmonostearat | 840.0 mg |
| | 2000.0 mg |

## Patentansprüche

1. Verbindungen der allgemeinen Formel **I** 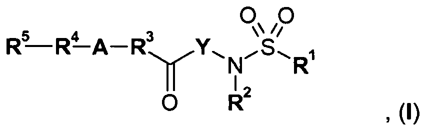 **dadurch gekennzeichnet, dass** sie aus folgenden Verbindungen ausgewählt sind:
| **Beispiel** | **Struktur** |
|---|---|
| (1) | 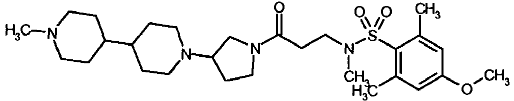 |
| (2) | 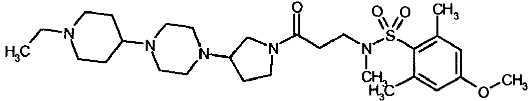 |
| (3) | 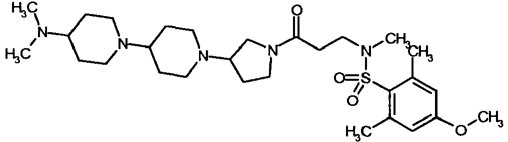 |
| (4) | 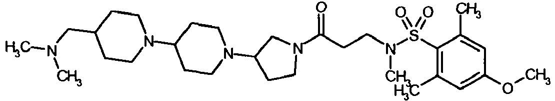 |
| (5) | 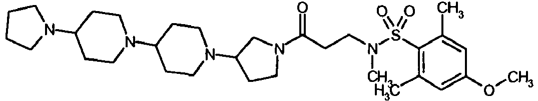 |
| (6) | 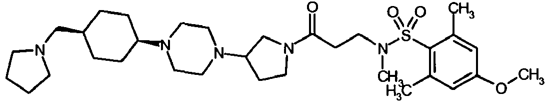 |
| (7) | 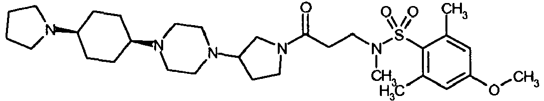 |
| (8) | 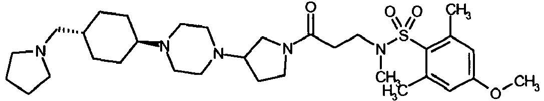 |
| (9) | 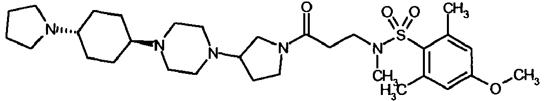 |
| (10) | 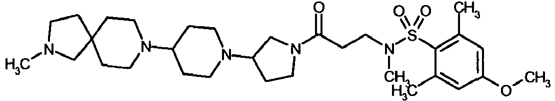 |
| (11) | 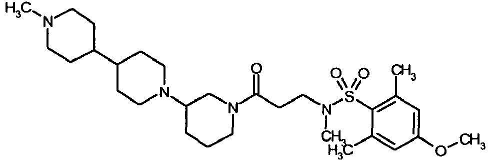 |
| (12) | 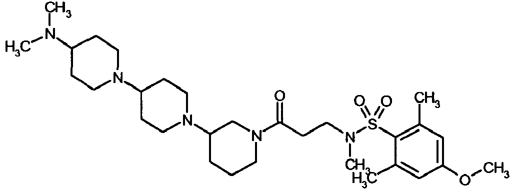 |
deren Enantiomere, deren Diastereomere, deren Gemische und deren Salze, insbesondere deren physiologisch verträgliche Salze mit organischen oder anorganischen Säuren oder Basen.

2. Physiologisch verträgliche Salze der Verbindungen nach Anspruch 1 mit anorganischen oder organischen Säuren oder Basen.

3. Arzneimittel, enthaltend eine Verbindung nach Anspruch 1 oder ein physiologisch verträgliches Salz gemäß Anspruch 2 neben gegebenenfalls einem oder mehreren inerten Trägerstoffen und/oder Verdünnungsmitteln.

4. Verwendung einer Verbindung nach mindestens einem der Ansprüche 1 bis 2 zur Herstellung eines Arzneimittels zur akuten und prophylaktischen Behandlung von akuten Schmerzen, Eingeweideschmerzen, neuropathischen Schmerzen, entzündlichen / Schmerzrezeptor-vermittelten Schmerzen, Tumorschmerzen und Kopfschmerz-Erkrankungen.

5. Verfahren zur Herstellung eines Arzneimittels gemäß Anspruch 3, **dadurch gekennzeichnet, dass** auf nichtchemischem Weg eine Verbindung nach mindestens einem der Ansprüche 1 bis 2 in einen oder mehrere inerte Trägerstoffe und/oder Verdünnungsmittel eingearbeitet wird.

## Claims

1. Compounds of general formula I 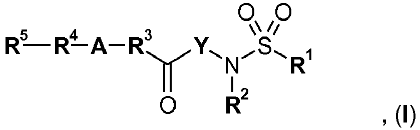 **characterised in that** they are selected from among the following compounds:
| **Example** | **Structure** |
|---|---|
| (1) | 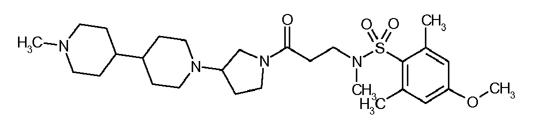 |
| (2) | 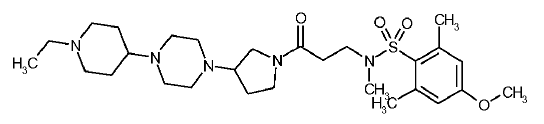 |
| (3) | 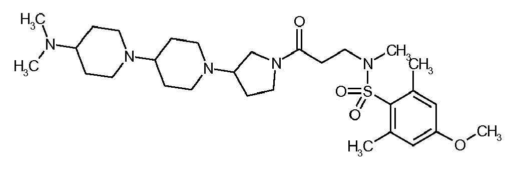 |
| (4) | 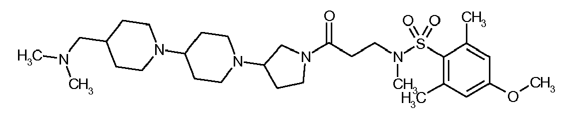 |
| (5) | 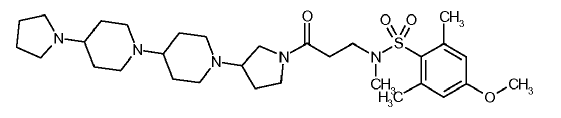 |
| (6) | 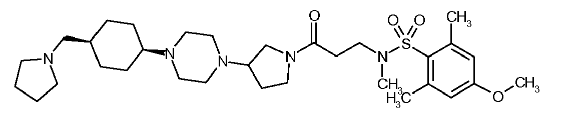 |
| (7) | 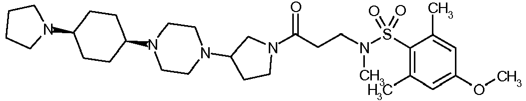 |
| (8) | 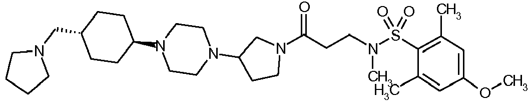 |
| (9) | 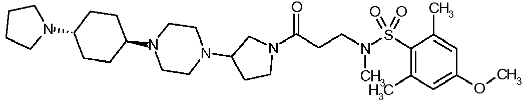 |
| (10) | 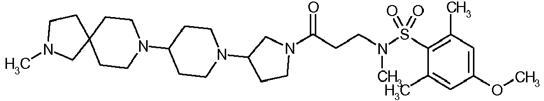 |
| (11) | 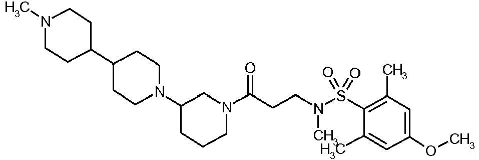 |
| (12) | 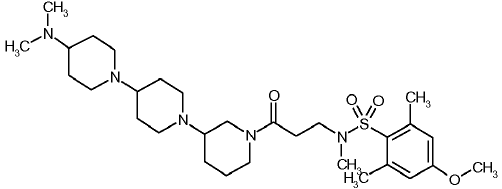 |
the enantiomers, the diastereomers, the mixtures thereof and the salts thereof, particularly the physiologically acceptable salts thereof with organic or inorganic acids or bases.

2. Physiologically acceptable salts of the compounds according to claim 1 with inorganic or organic acids or bases.

3. Medicaments containing a compound according to claim 1 or a physiologically acceptable salt according to claim 2, optionally together with one or more inert carriers and/or diluents.

4. Use of a compound according to at least one of claims 1 to 2 for preparing a medicament for the acute and prophylactic treatment of acute pain, visceral pain, neuropathic pain, inflammatory/pain receptor-mediated pain, tumour pain and headache diseases.

5. Process for preparing a medicament according to claim 3, **characterised in that** a compound according to at least one of claims 1 to 2 is incorporated in one or more inert carriers and/or diluents by a non-chemical method.

## Revendications

1. Composés de formule générale I 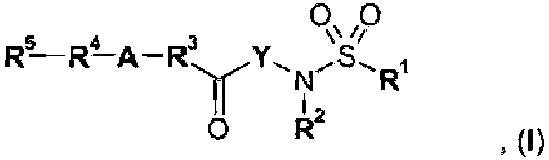 **caractérisés en ce qu'**ils sont choisis parmi les composés suivants :
| Exemple | structure |
|---|---|
| (1) | 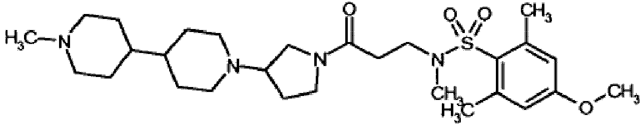 |
| (2) | 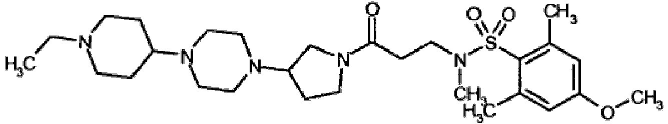 |
| (3) | 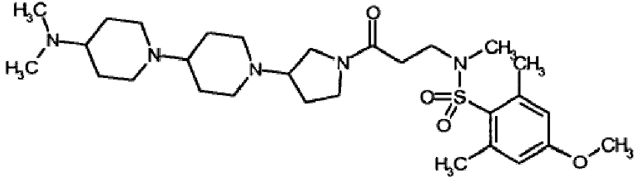 |
| (4) | 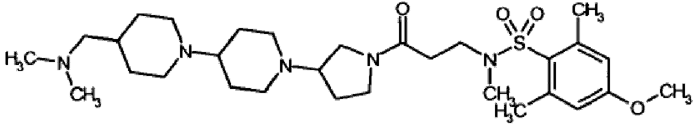 |
| (5) | 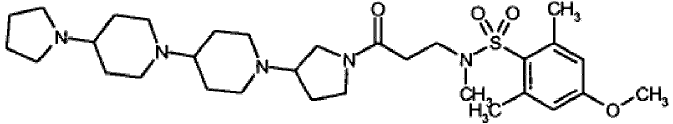 |
| (6) | 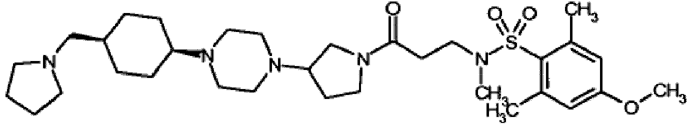 |
| (7) | 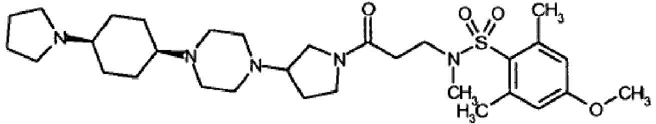 |
| (8) | 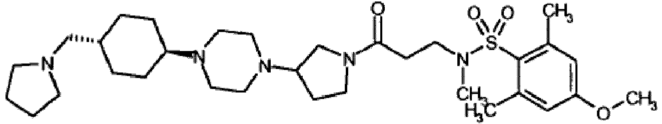 |
| (9) | 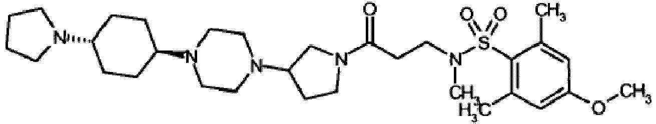 |
| (10) | 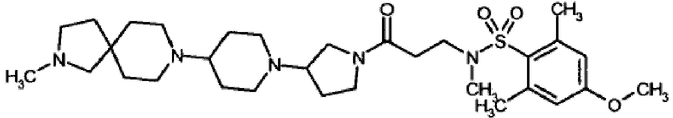 |
| (11) | 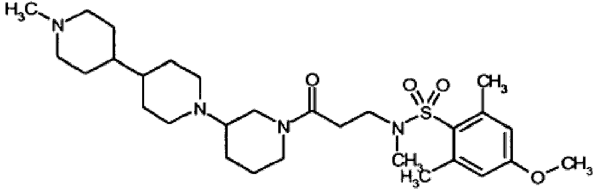 |
| (12) | 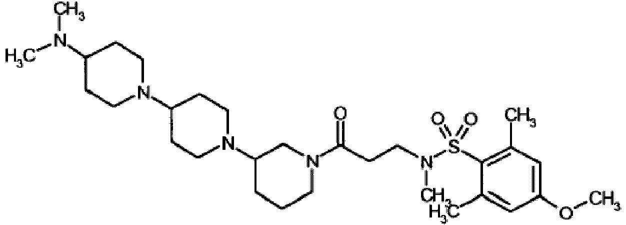 |
leurs énantiomères, leurs diastéréomères, leurs mélanges et leurs sels, en particulier, leurs sels physiologiquement acceptables avec des acides ou des bases organiques ou anorganiques.

2. Sels physiologiquement acceptables des composés selon la revendication 1, avec des acides ou des bases inorganiques ou organiques.

3. Médicament contenant un composé selon la revendication 1, ou sel physiologiquement acceptable selon la revendication 2, outre éventuellement un ou plusieurs véhicules et/ou diluants inertes.

4. Utilisation d'un composé selon au moins l'une des revendications 1 à 2, pour la production d'un médicament pour le traitement aigu et prophylactique de douleurs aiguës, de douleurs viscérales, de douleurs neuropathiques, de douleurs inflammatoires médiées par des récepteurs nociceptifs, de douleurs tumorales et de céphalées.

5. Procédé de production d'un médicament selon la revendication 3, **caractérisé en ce que**, de façon non chimique, un composé selon au moins l'une des revendications 1 à 2 est intégré dans un ou plusieurs véhicules et/ou diluants inertes.
